# DEMANDE DE BREVET EUROPEEN

(11) **EP 4 160 612 A2**
(43) Date de publication de la demande: **05.04.2023**
(21) Numéro de dépôt: 22197368.8
(22) Date de dépôt: 23.09.2022
(51) Int. Cl.: G16H 40/60, G16H 30/20, A41D 1/00, A61B 5/00, A61B 8/00, B25J 9/00

(54) **DISPOSITIF MÉDICAL PORTABLE**

(30) Priorité: 29.09.2021 FR 2110272
(71) Demandeur: SUPERSONIC IMAGINE, 13290 Aix-en-Provence (FR)
(72) Inventeur: LERAT, Damien, 13290 Aix-en-Provence (FR)
(74) Mandataire: Paustian & Partner Patentanwälte mbB

(57) **Abrégé**

La présente divulgation concerne un dispositif médical portable comprenant :
un équipement médical d'acquisition de données et un système porteur, dans lequel l'équipement médical comprend :
une unité de traitement configurée pour traiter les données acquises par une sonde associée avec l'unité de traitement,
et dans lequel le système porteur est configuré pour équiper le corps d'un utilisateur du dispositif avec l'équipement médical.

## Description

### Domaine Technique

La présente invention est relative aux systèmes médicaux et aux procédés utilisant un tel système. Un tel système peut constituer un système d'examen médical. Plus particulièrement, il peut constituer un dispositif à ultrasons.

### Technique antérieure

Un système de visualisation médical comprend habituellement des moyens électroniques, par exemple un capteur et/ou une sonde (par exemple une sonde d'échographie) pour acquérir des données d'un patient, et un processeur pour traiter des données acquises. Il peut comprendre en outre un module de pilotage pour piloter le système, associé notamment à une interface d'utilisateur. Le dispositif médical peut être destiné à fournir des données d'un milieu à examiner, affichées sur un écran. Dans le cas des applications médicales, le milieu est un corps, par exemple une partie du corps d'un patient (muscles, foetus, sein, foie, abdomen, ...). En général, la sonde est maintenue contre la surface d'un milieu observé, afin d'acquérir des données sur ce milieu.

Souvent l'utilisateur du dispositif médical regarde en même temps sur un écran du dispositif. Cependant, l'écran n'est généralement pas directement adjacent au milieu. Par exemple, dans le cas de l'examen d'un patient allongé sur un lit ou assis sur une chaise, l'écran est souvent placé sur un chariot (ou plus généralement une plate-forme) placé à côté du lit ou de la chaise et donc (du point de vue de l'utilisateur) dans une direction complètement différente de celle du milieu. Par conséquent, l'utilisateur du dispositif médical est obligé de prendre une position inconfortable et non naturelle pendant l'acquisition des données. L'utilisateur est notamment obligé de tourner la tête et généralement aussi tout le torse.

Dans ce contexte, la littérature internationale montre que 80 à 90 % des échographistes ressentent des douleurs lors de l'exécution de scans à un moment ou à un autre de leur carrière. Parmi eux, environ 20 % subiront une blessure qui changera leur carrière ou leur vie.

On connaît différents types de dispositifs médicaux. Un dispositif médical, par exemple un dispositif à ultrasons classique se présente sous la forme susmentionnée d'un chariot à ultrasons à roues (ou plus généralement d'une plate-forme à ultrasons) qui est configuré pour être déplacé au sol. Le chariot est configuré pour supporter les parties relativement lourdes du dispositif médical, en particulier son unité de traitement et son ou ses dispositifs d'écran. De tels dispositifs peuvent avoir une qualité d'image relativement bonne et être polyvalents (en raison de l'utilisation possible de plusieurs sondes des types différentes), mais ils manquent de maniabilité. De plus, ces types de dispositifs ont généralement besoin d'une source d'alimentation principale.

Il a également été proposé d'intégrer complètement le dispositif médical au siège ou au lit d'examen. Cependant, ces systèmes sont généralement relativement lourds et leur maniabilité est encore moins bonne.

Par ailleurs, il existe des dispositifs à ultrasons portables qui utilisent par exemple un smartphone ou une tablette électronique pour l'imagerie. La sonde communique dans ce cas avec le smartphone ou la tablette. Ces systèmes peuvent être facilement déplacés. Cependant, ils ne comprennent généralement qu'une seule sonde. En outre, ils ont un écran relativement petit et une autonomie limitée en raison d'une batterie relativement petite. En raison des pièces relativement compactes et légères d'un tel système et de l'autonomie réduite, la qualité de l'image est généralement aussi moins bonne que celle d'un dispositif à ultrasons classique. En outre, ils doivent toujours être portés à la main, ce qui empêche l'utilisateur d'utiliser ses mains pour d'autres tâches et ce qui peut propager des infections nosocomiales.

### Exposé de l'invention

La présente divulgation a donc pour but de prévoir un dispositif médical portable qui remédie les inconvénients susmentionnés. En particulier, l'objectif est de fournir un dispositif médical portable avec une flexibilité, autonomie, et polyvalence optimisées. En outre, il est souhaitable que le dispositif médical permette à l'utilisateur du dispositif d'éviter les positions inconfortables et/ou non naturelles pendant l'acquisition des données et de libérer les mains de la tenue du dispositif.

La présente divulgation se rapporte à un dispositif médical portable comprenant :
un équipement médical d'acquisition de données et un système porteur, dans lequel l'équipement médical comprend :
une unité de traitement configurée pour traiter les données acquises par une sonde associée avec l'unité de traitement,
et dans lequel le système porteur est configuré pour équiper le corps d'un utilisateur du dispositif avec l'équipement médical.

En fournissant un tel dispositif médical, l'utilisateur peut être en mesure de porter (ou s'équiper avec) le système porteur qui permet un haut degré de maniabilité de l'utilisateur. En d'autres termes, l'utilisateur peut par exemple se déplacer en ayant toujours l'équipement médical avec lui/elle. Dans ce contexte, différents éléments de l'équipement médical (décrits plus loin) peuvent par exemple être disposés sur le dispositif médical de telle sorte qu'ils soient toujours dans une position appropriée pour l'utilisateur, indépendamment de sa position. Ainsi, l'utilisateur n'est par exemple pas obligé de prendre des positions inconfortables pour visualiser les informations pendant l'examen et les éléments nécessaires à l'examen (par exemple une sonde) peuvent toujours être facilement accessibles. Comme ces éléments peuvent être maintenus par le système porteur, l'utilisateur peut même avoir les mains libres lorsqu'il ne manipule pas les éléments. En outre, le système de support peut aider à supporter le poids de l'équipement médical.

Le système porteur peut être configuré pour être vêtu et/ou endossé par l'utilisateur du dispositif.

Le dispositif médical peut être un dispositif à ultrasons. Ainsi, l'équipement médical d'acquisition de données peut être un l'équipement à ultrasons et/ou la sonde une sonde à ultrasons.

L'équipement médical peut comprendre en outre la sonde. La sonde peut être une sonde à ultrasons.

L'unité de traitement peut comprendre une batterie et/ou peut être configurée pour fonctionner de manière autonome à partir d'une alimentation électrique externe.

Le dispositif médical peut comprendre en outre une unité de commande avec une interface utilisateur configurée pour permettre à l'utilisateur de commander le dispositif médical.

L'unité de traitement peut être configurée pour recevoir et/ou traiter des données externes d'un système externe. Par exemple, ces données externes peuvent comprendre des données médicales acquises par un autre dispositif, par exemple des données d'imagerie par résonance magnétique (IRM) et/ou des données de tomographie par ordinateur (CT) et/ou des données acquises par un autre dispositif à ultrasons. L'unité de traitement peut comprendre une interface de communication par exemple sans fil, pour recevoir les données externes et/ou transmettre des données traitées vers/depuis un système externe.

L'équipement médical peut comprendre en outre un écran d'affichage et/ou un casque, par exemple de réalité augmentée. L'unité de traitement peut être configurée pour que le dispositif d'affichage et/ou le casque affiche des informations basées sur les données traitées et/ou les données externes.

Le système porteur peut être configuré pour être ajusté au corps de l'utilisateur et/ou pour être installé contre/sur le corps de l'utilisateur.

Le système porteur peut comprendre un sac à dos configuré pour porter l'unité de traitement.

Le sac à dos peut comprendre un système de ceinture configuré pour être porté par le tronc de l'utilisateur et pour faire supporter au tronc de l'utilisateur au moins une partie du poids du système porteur et/ou de l'équipement médical.

Le sac à dos et/ou le système de ceinture peut être configuré pour porter la batterie et/ou l'unité de contrôle et/ou des accessoires configurés pour être utilisable en préparation et/ou pendant un examen médical. Ces accessoires peuvent comprendre par exemple un lecteur code d'identifiant (par exemple pour identifier un marqueur ou un kit de biopsie), une protection de l'équipement médical, des capteurs, et/ou des gants. Le lecteur code d'identifiant peut être par exemple un lecteur de code barres ou de QR codes, ou de puces RFID.

Le système porteur peut comprendre un premier bras configuré pour maintenir la sonde (et/ou un ou plusieurs accessoires) dans une position telle que la sonde puisse être saisie par une main de l'utilisateur.

Le premier bras peut comprendre un porte sonde configuré pour tenir la sonde.

Le premier bras peut être en outre configuré pour tenir et/ou contenir un câble reliant la sonde à l'unité de traitement.

Le câble peut être extensible hors du sac à dos et/ou hors du premier bras.

Au moins un élément parmi le premier bras, le deuxième bras, et le porte sonde peut être motorisé.

L'unité de traitement peut être configuré pour contrôler un déplacement du premier et/ou deuxième bras et/ou du porte sonde d'une manière automatique.

Le premier bras et/ou le porte sonde peut comprendre au moins un capteur de localisation.

L'unité de traitement peut être configurée pour déterminer un déplacement de la sonde en fonction des données acquises par le ou les capteurs de localisation.

En outre, l'unité de traitement peut être configurée pour fusionner des données acquises par la sonde avec des données externes, en particulier en fonction des données acquises par le capteur de localisation.

Finalement, au moins un élément parmi le premier bras, le deuxième bras, et l'unité de traitement peut être fixée de manière réversible sur le système de porteur afin de s'adapter à la latéralité de l'utilisateur (selon que l'utilisateur est gaucher ou droitier).

Le premier bras peut être mobile et/ou flexible, de telle sorte que la position déployée de la sonde soit réglable.

Le premier bras peut être mobile de telle sorte que la sonde peut être déplacée de la position étendue à une position rétractée, dans laquelle la sonde peut être plus proche et/ou adjacente à au moins un élément parmi le corps de l'utilisateur, le sac à dos et/ou le système de ceinture.

Le système porteur peut comprendre un deuxième bras configuré pour maintenir le dispositif d'affichage dans une position étendue par rapport à l'utilisateur, de sorte que les informations affichées puissent être accessibles à l'utilisateur et/ou au patient.

Le deuxième bras peut être mobile de sorte que la position du dispositif d'affichage peut être ajustée.

Le deuxième bras peut être mobile de sorte que le dispositif d'affichage peut être déplacé de la position étendue à une position rétractée, où le dispositif d'affichage peut être plus proche et/ou adjacent à au moins un élément parmi le corps de l'utilisateur, le sac à dos et le système de ceinture.

Le premier et/ou le deuxième bras peut être fixé de manière mobile au sac à dos et/ou au système de ceinture.

Le premier et/ou le deuxième bras peut être configuré pour être maintenu par le sac à dos et/ou le système de ceinture.

Le système porteur peut comprendre un ou deux pieds et/ou jambes configurés pour supporter au moins une partie du poids du système porteur et/ou du dispositif médical sur un sol sur lequel se tient l'utilisateur. Le ou les jambes peuvent être rétractables. Le système porteur peut également être pourvu de trois jambes, utilisables simultanément ou alternativement, afin d'assurer une stabilité optimale a l'utilisateur, en particulier lorsque le sol présente des dénivelés importants. La ou les jambes permettent ainsi des interventions sur zones très accidentées.

Le au moins un pied et/ou jambe peut être configuré pour être articulé et/ou mobile de sorte que l'utilisateur peut se mouvoir et/ou marcher (et/ou se reposer du poids du système porteur), tandis que le pied et/ou jambe supporte au moins une partie du poids du système porteur et/ou de l'équipement médical sur le sol.

Le au moins un pied et/ou jambe peut être configuré pour être associé aux membres inférieurs de l'utilisateur pour suivre le mouvement des membres inférieurs de l'utilisateur.

Le système porteur peut comprendre ou former un exosquelette.

L'exosquelette peut comprendre au moins l'un des éléments suivants : le sac à dos, le système de ceinture, le premier bras, le deuxième bras, et le au moins un pied et/ou jambe.

Les caractéristiques et avantages de l'invention apparaitront à la lecture de la description, donnée uniquement à titre d'exemple non limitatif, et faite en référence aux figures annexées. Notamment, les exemples illustrés dans les figures peuvent être combinés sauf incohérence notoire.

### Brève description des figures

[Fig. 1a] montre schématiquement une vue de face d'un premier mode de réalisation d'un dispositif selon la présente divulgation dans une position debout.
[Fig. 1b] montre schématiquement une vue latérale du dispositif de la figure 1a.
[Fig. 2a] montre schématiquement une vue de face du dispositif des figures 1a et 1b dans une position assise.
[Fig. 2b] montre schématiquement une vue latérale du dispositif de la figure 2a.
[Fig. 3a] montre de manière schématique une vue de face du dispositif des figures 1a, 1b et 2a, 2b dans une position de déplacement et/ou de stationnement.
[Fig. 3b] montre schématiquement une vue latérale du dispositif de la figure 3a.
[Fig. 4] montre schématiquement une vue de face d'un deuxième mode de réalisation d'un dispositif selon la présente divulgation comprenant un visiocasque (ou casque de réalité virtuelle) et une interface utilisateur.
[Fig. 5] montre de manière schématique une vue de face d'un troisième mode de réalisation d'un dispositif selon la présente divulgation comprenant des accessoires.
[Fig. 6a] montre schématiquement une vue de face d'un quatrième mode de réalisation d'un dispositif selon la présente divulgation.
[Fig. 6b] montre schématiquement une vue latérale du dispositif de la figure 6a avec des modifications optionnelles.
[Fig. 7a] montre de manière schématique une vue de face d'un cinquième mode de réalisation d'un dispositif selon la présente divulgation.
[Fig. 7b] montre de manière schématique une vue latérale du dispositif de la figure 7a avec des modifications optionnelles.
[Fig. 8a] montre de manière schématique une vue de face d'un sixième mode de réalisation d'un dispositif selon la présente divulgation.
[Fig. 8b] montre de manière schématique une vue latérale du dispositif de la figure 8a.

### Description des modes de réalisation

Sur les différentes figures, fournies à titre d'illustration, les mêmes références numériques désignent des éléments identiques ou similaires. Les différents modes de réalisation présentés dans les figures peuvent être combinés sauf incohérence notoire.

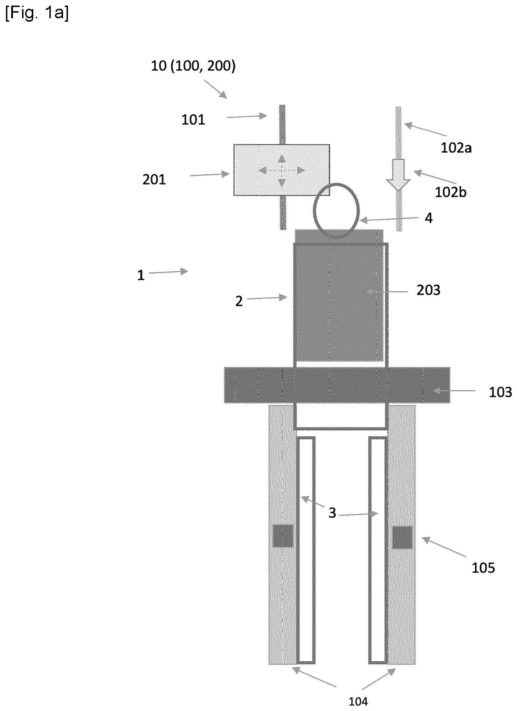

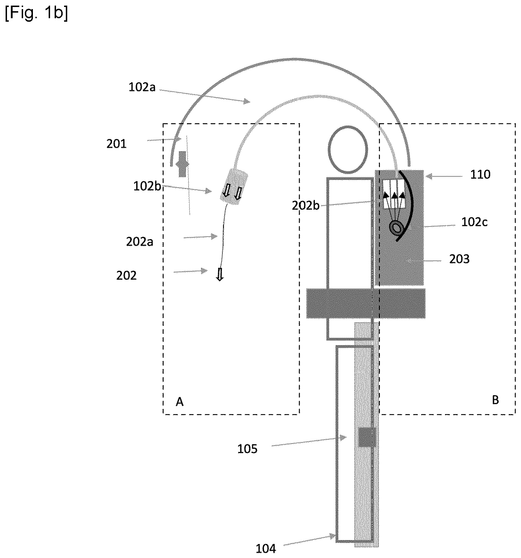

La figure 1a montre schématiquement une vue de face d'un premier mode de réalisation d'un dispositif 10 selon la présente divulgation dans une position debout. La figure 1b représente schématiquement une vue latérale du dispositif de la figure 1a. Le dispositif ultrasonique portable 10 peut comprendre un système porteur 100 et un équipement médical 200. L'équipement médical peut comprendre une unité de traitement 203 configurée pour traiter les données acquises par une sonde 202 associée à l'unité de traitement. La sonde peut également faire partie du dispositif médical portable 10, notamment de l'équipement médical 200, ou constituer un élément extérieur. La sonde peut être une sonde à ultrasons. En d'autres termes, la sonde peut comprendre un ou plusieurs transducteurs ultrasonores.

Toutefois, la sonde peut également être configurée pour utiliser d'autres technologies que la technique d'acquisition ultrasonore. Par exemple, elle peut être ou comprendre d'autres types de capteurs ou de techniques d'acquisition de données combinées. Dans un exemple, la sonde peut comprendre un ou plusieurs capteurs et/ou transducteurs optiques et/ou lasers. Il est également possible que la sonde soit configurée pour une acquisition de données opto-acoustique. En conséquence, l'unité de traitement peut être configurée pour traiter également d'autres types de données que les données ultrasonores. Par ailleurs, outre la sonde, le dispositif peut également être configuré pour collaborer avec d'autres types de dispositifs médicaux, par exemple un ou plusieurs dispositifs d'intervention (par exemple un dispositif de cryoablation, un marqueur et/ou un dispositif configuré pour retirer de la matière du milieu, comme par exemple une aiguille de biopsie ou tout instrument chirurgical utilisé en corrélation avec le dispositif). En conséquence, le dispositif 10 peut être configuré pour comprendre ou être utilisé avec toute combinaison de ces sondes, capteurs et dispositifs médicaux.

Le système porteur 100 peut être configuré pour équiper le corps d'un utilisateur 1 avec l'équipement échographique 200. En particulier, le système porteur peut être configuré pour s'adapter au corps de l'utilisateur et/ou pour être monté sur/contre le corps/tronc de l'utilisateur. Par exemple, le système de support 100 peut être configuré pour être porté par un utilisateur. Le système porteur 100 peut notamment comprendre ou se présenter sous la forme d'un exosquelette. Par conséquent, l'utilisateur peut être capable de se déplacer (par exemple marcher et/ou s'asseoir) lorsqu'il est équipé du système porteur 100.

Par exemple, le système porteur 100 peut comprendre un sac à dos 110 configuré pour porter l'unité de traitement 203. Le sac à dos 110 peut comprendre un système de ceinture 103 configuré pour être porté par le tronc de l'utilisateur et pour amener le tronc de l'utilisateur à supporter au moins une partie du poids du système porteur 100 et/ou de l'équipement médical 200. Toutefois, le système de ceinture 203 peut également être un élément du système porteur, qui est distinct du sac à dos 110.

Dans un exemple, le système de ceinture peut comprendre une ceinture configurée pour entourer la hanche de l'utilisateur ou pour être portée par la hanche de l'utilisateur. Le système de ceinture peut également comprendre une ou plusieurs sangles, par exemple sous la forme de sangles de sac à dos.

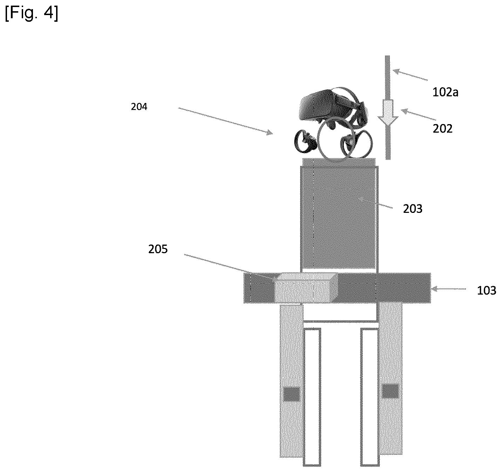

Le système de ceinture peut aussi être configuré à la manière d'un baudrier. Dans ce cas, le système de ceinture ou le système porteur peut être peu encombrant, assez léger et adapté individuellement à la morphologie de l'utilisateur. Le système de sac à dos et/ou de ceinture peut être configuré pour porter une batterie et/ou une unité de commande avec une interface utilisateur (cf. par exemple l'unité de commande 205 sur la figure 4). L'unité de commande peut permettre à l'utilisateur ou un collaborateur de contrôler l'équipement médical.

La batterie peut être configurée pour alimenter le dispositif 10, en particulier l'équipement médical 200, en énergie électrique. Ainsi, le dispositif mobile 100 et en particulier son équipement médical peut être configuré pour fonctionner de manière autonome à partir d'une alimentation électrique externe. Ainsi, l'utilisateur peut se déplacer sans être gêné par un quelconque câble d'alimentation électrique.

Le système porteur 100 peut comprendre un premier bras 102a configuré pour maintenir la sonde 202 dans une position telle que la sonde peut être facilement et directement saisie par une main de l'utilisateur. Par exemple, le premier bras peut être agencé de manière à s'étendre au-dessus d'une épaule et/ou de la tête 4 de l'utilisateur 1, en particulier du dos de l'utilisateur vers le côté avant. Par exemple, le bras peut être fixé au sac à dos 110 et s'étendre depuis celui-ci vers le côté avant de l'utilisateur, où la sonde est maintenue par le premier bras.

Le premier bras 102a peut être en outre configuré pour tenir et/ou contenir un câble 202a reliant la sonde 202 à l'unité de traitement 203, en particulier un connecteur 202b de l'unité de traitement 203. Le premier bras peut comprendre un porte sonde 102b, par exemple à son extrémité avant (ou extrémité saillante) qui est configurée pour maintenir la sonde 202. Le porte sonde peut être configuré pour recevoir et/ou fixer une ou plusieurs sondes et/ou des accessoires.

Le premier bras peut être mobile de sorte que la position déployée de la sonde 202 est réglable.

Le premier et/ou deuxième bras peut par exemple être réalisé dans un matériau présentant une certaine souplesse, un système de ressorts ou de pièces mécaniques, il peut être élastique et/ou pliable (par exemple un plastique ou un polycarbonate). En même temps, le premier et/ou deuxième bras peut être suffisamment rigide pour supporter le poids de la sonde et/ou du câble. Le premier et/ou deuxième bras peut également comprendre une ou plusieurs charnières (en particulier, si le bras est réalisé dans un matériau rigide, par exemple un métal).

Généralement le premier et/ou deuxième bras peut être développé de différentes manières, en utilisant des propriétés différentes :
Selon un premier exemple, le premier et/ou deuxième bras peut être configuré pour permettre une déformation élastique. Par exemple, le premier et/ou deuxième bras peut comprendre ou être constitué d'un matériau qui se plie et reprend sa forme initiale, par exemple du titane, fibre de verre, métal (acier). L'élasticité peut être provoquée par exemple par la structure du bras (p.ex. par une forme de ressort) et/ou par les propriétés du matériau.

Selon un deuxième exemple, le premier et/ou deuxième bras peut comprendre des propriétés flexibles : par exemple, le premier et/ou deuxième bras peut comprendre ou être constitué d'acier ou de plastique. La propriété flexible peut être apportée par la structure en col de cygne flexible (« flexible gooseneck tube » en anglais). Ce type de dispositif peut être dirigé à l'aide de câble(s).

Selon un troisième exemple, le premier et/ou deuxième bras peut comprendre des propriétés rigides : par exemple, le premier et/ou deuxième bras peut comprendre des segments rigides en carbone, aluminium, plastique, reliés par des articulations (rotules) supportées par exemple par des vérins et/ou des ressorts. Ces articulations peuvent être actionnées à l'aide de moteurs électriques, de vérin(s) par exemple hydrauliques, de câbles.

Selon un quatrième exemple, le premier et/ou deuxième bras peut comprendre des propriétés d'une flexibilité associée à de la mémoire de forme : Par exemple, le premier et/ou deuxième bras peut comprendre des propriétés d' alliages à mémoire de forme (simple mémoire ou double mémoire).

Notamment, selon le quatrième exemple, le premier et/ou deuxième bras peut comprendre des propriétés d'un tube plein ou creux composé d'un alliage à mémoire de forme dont la section est suffisante pour supporter le poids des équipements. L'alliage peut par exemple être du nitinol (alliage de nickel et titane).

En outre, selon le quatrième exemple, le premier et/ou deuxième bras peut comprendre des propriétés d'un système chauffant et refroidissant adjacent au tube ou à l'intérieur de celui-ci, permettant de replier le bras en position rangée et/ou de le déployer. Le pourcentage de nickel par rapport du titane peut être sélectionné pour gérer la température à laquelle le bras change sa forme.

Les exemples ci-dessus peuvent aussi être combinés à volonté. Par exemple, un col de cygne peut être utilisé en complément de l'assemblage rigide de manière à créer un doigt à l'extrémité du bras. En outre, des ressorts à mémoire de forme peuvent être utilisés en lieu et place de câbles utilisés pour motoriser le col de cygne, et/ou pour remplacer des vérins dans les assemblages rigides. Par ailleurs, des matériaux flexibles peuvent être utilisés pour éviter de blesser l'utilisateur et le patient et/ou pour rendre l'examen plus confortable lorsque le bras est motorisé. En outre, le premier et/ou deuxième bras et/ou le porte sonde peut être motorisé.

Par exemple, le premier bras peut comprendre un ou plusieurs actionneurs (par exemple des actionneurs électromécaniques (et/ou des alliages à mémoire de forme), comme par exemple un moteur) configurés pour déplacer automatiquement le bras. L'unité de traitement ou toute unité de commande externe peut être configurée pour commander le mouvement du bras. Ainsi, le bras peut être commandé pour effectuer un mouvement spécifique, par exemple mise à disposition de la sonde et/ou guide des mouvements de la sonde pendant l'acquisition de données.

Par ailleurs, le premier bras et/ou le porte sonde peut comprendre un capteur de localisation. Dans ce cas, l'unité de traitement peut être configurée pour déterminer un déplacement de la sonde (par exemple par rapport du milieu) en fonction des données acquises par le capteur de localisation. En outre, l'unité de traitement peut être configurée pour fusionner des données acquises par la sonde avec des données externes en fonction des données acquises par le capteur de localisation. Ces données externes peuvent par exemple comprendre des données médicales acquises auparavant, par exemple des données d'échographie, de tomographie et/ou d'IRM. Ainsi, il devient possible que le bras balaye une position spécifique du milieu ou se déplace sur une zone spécifique du milieu pendant l'acquisition des données, tel que des données soient acquises pour la même zone du milieu que celle concernée par les données externes.

En variante, le dispositif 10 peut comprendre une interface utilisateur (par exemple un ou plusieurs capteurs positionnés au niveau du bras) configurée pour permettre à l'utilisateur de contrôler le mouvement du bras. L'utilisateur peut par exemple recevoir sur l'écran des informations de guidage du dispositif configurées pour guider un mouvement de la sonde. Ces informations de guidage peuvent être déterminées par l'unité de traitement en fonction des données acquises par le capteur de localisation et données externes, afin de permettre une fusion des données acquises par la sonde avec des données externes.

Le capteur de localisation peut utiliser une technologie de triangulation en radio et/ou un ou plusieurs « MEMS » (systèmes micro électromécaniques) équipant le premier bras et/ou le porte sonde.

En outre, le câble 202a peut être extensible hors du sac à dos 110 et/ou hors du premier bras 102a (et par exemple hors du porte-sonde 102b). Le dispositif 10, notamment son système porteur 100, peut par exemple comprendre un ou plusieurs enrouleurs 102c configurés pour permettre l'extension ou le déroulement du câble 202a. Optionnellement, le dispositif, en particulier la bobine 102c, peut être configuré pour rétracter automatiquement un câble déployé.

En conséquence, du fait de la possibilité d'étendre le câble de la sonde et/ou de déplacer le premier bras, la sonde peut être déplacée par l'utilisateur dans toute position appropriée, lors par exemple de l'acquisition de données d'un milieu via la sonde.

Optionnellement, le dispositif peut être configuré pour comprendre ou être utilisé avec une pluralité de sondes. Dans ce cas, le dispositif peut comprendre respectivement une pluralité de bobines 102c. En outre, le porte sonde peut être configuré pour recevoir une pluralité de sondes. De même, l'unité de traitement 203 peut comprendre une pluralité de connecteurs 202b et/ou le bras 102a peut être configuré pour tenir et/ou contenir une pluralité de câbles 202a.

Il est en outre noté qu'au moins une sonde ou toutes les sondes peuvent collaborer et/ou communiquer avec l'unité de traitement sans fil, c'est-à-dire sans utiliser de câble. Dans ce cas, le porte sonde 102b peut être configuré pour maintenir les sondes de manière détachable, lorsqu'elles ne sont pas. En conséquence, l'utilisateur peut saisir et détacher une sonde, lorsque la sonde lui est nécessaire.

Le premier bras peut être mobile de sorte que la sonde peut être déplacée de la position étendue à une position rétractée, dans laquelle la sonde peut être plus proche et/ou adjacente à au moins un élément parmi le corps de l'utilisateur, le sac à dos et le système de ceinture.

Le dispositif médical 10, en particulier l'équipement médical 200, peut en outre comprendre un dispositif d'écran 201. Il est également possible que le dispositif d'écran soit un élément externe par rapport à l'équipement échographique, et que par exemple l'équipement échographique comprenne (uniquement) la sonde 202. Le dispositif d'écran peut être configuré pour afficher des informations basées sur les données traitées par l'unité de traitement 203. Par exemple, le dispositif d'écran peut être connecté à l'unité de traitement 203 via un câble et/ou via une interface de communication sans fil. En outre, le dispositif d'écran peut être configuré pour afficher d'autres informations. Ces informations supplémentaires peuvent comprendre par exemple des informations concernant un patient (par exemple un dossier de patient), des données médicales d'un patient acquises dans le passé (par exemple des données échographiques et/ou d'autres données acquises par un autre dispositif, par exemple des données d'imagerie par résonance magnétique (IRM) et/ou des données de tomographie par ordinateur (CT)).

Le dispositif d'écran peut par exemple comprendre un écran tactile. En conséquence, le dispositif d'écran peut être configuré pour permettre à l'utilisateur de contrôler le dispositif 10 via l'écran tactile. Toutefois, le dispositif 10 peut comprendre tout autre interface utilisateur, par exemple au niveau du système de ceinture 103 (cf. par exemple fig. 4).

Le dispositif à écran 201 peut être maintenu par un deuxième bras 101. En particulier, le système porteur 100 peut comprendre le deuxième bras 101. Ledit bras peut être configuré pour maintenir le dispositif d'écran 201 dans une position étendue par rapport à l'utilisateur, de sorte que les informations affichées puissent être visibles pour l'utilisateur. Par exemple, le deuxième bras peut être agencé de manière à s'étendre sur une épaule et/ou au-dessus de la tête 4 de l'utilisateur 1.

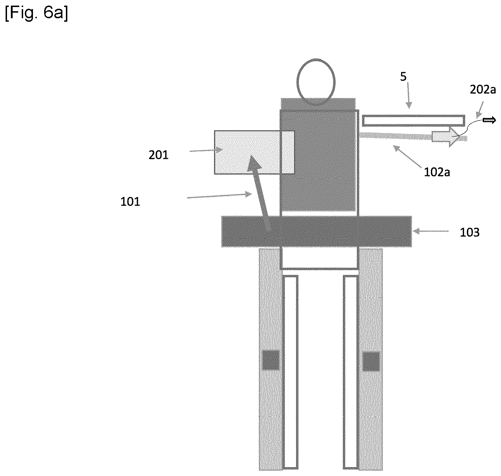

En général, une zone A située devant l'utilisateur peut être appelée la zone d'intervention (cf. fig. 1b). Le premier et/ou le second bras peut s'étendre vers cette zone d'intervention pour permettre à l'utilisateur de voir des informations sur l'écran et/ou d'utiliser l'équipement. La zone B derrière l'utilisateur peut être appelée zone de stockage. Le premier et/ou le second bras peut être fixé dans ou à proximité de la zone de stockage. De plus, une partie de l'équipement peut être rangée dans ou à proximité de la zone de stockage, notamment dans le sac à dos. En variante, le premier et/ou le second bras peut être fixé à la ceinture et/ou une partie de l'équipement peut être rangée sur la ceinture, comme décrit dans le contexte de la figure 6a, par exemple. Par exemple, le bras peut être attaché au sac à dos 110 et s'étendre depuis celui-ci vers le côté avant de l'utilisateur, où le dispositif d'écran 201 est tenu par le bras.

Dans un exemple, le premier bras 102a s'étend sur une épaule de l'utilisateur et le second bras 101 sur l'autre épaule. Cependant, ils peuvent également s'étendre sur la même épaule et/ou sur la tête de l'utilisateur 4.

Le deuxième bras 101 peut être mobile de façon à ce que la position du dispositif d'affichage puisse être ajustée. Comme décrit ci-dessus, le premier et le deuxième peuvent être sensiblement proches dans leur structure et/ou en ce qui concerne leur(s) matériau(x).

Par exemple, le deuxième bras peut être déplacé pour permettre un ajustement de l'orientation et/ou de la position de l'écran. Le deuxième bras peut en outre être configuré (par exemple au moyen d'une articulation) pour permettre à l'utilisateur de tourner le dispositif à écran, par exemple de manière à ce qu'un patient ou une autre personne se trouvant devant l'utilisateur puisse voir les informations affichées sur l'écran.

Le dispositif 10, en particulier le système porteur 100, peut en outre comprendre un ou deux pieds et/ou jambes 104 configurés pour supporter au moins une partie du poids du système porteur 100 et/ou de l'équipement médical 200 sur un sol sur lequel l'utilisateur 1 se tient debout. Le pied unique permet une position de repos pour l'utilisateur et/ou d'alléger la sensation de port de charge dans certaines configurations. Les pieds et/ou jambes peuvent être configurés pour être associés aux membres inférieurs de l'utilisateur afin de suivre le mouvement des membres inférieurs de l'utilisateur. Ce ou ces pieds ou jambes assurent également une meilleure stabilité du dispositif.

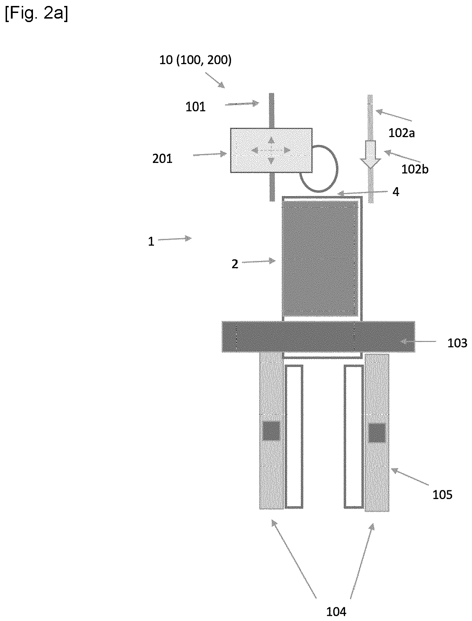

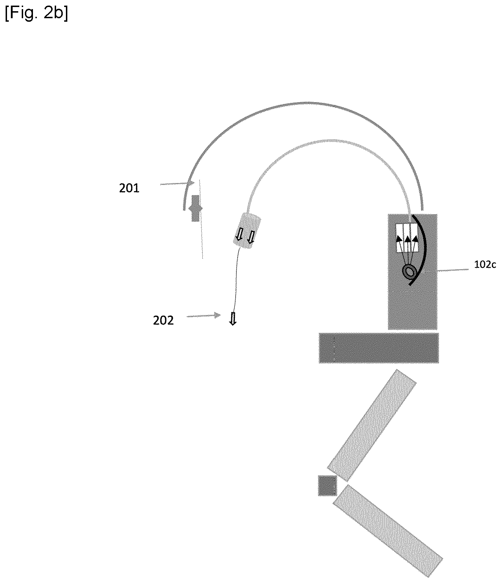

La figure 2a représente schématiquement une vue de face du dispositif des figures 1a et 1b dans une position assise. La figure 2b représente schématiquement une vue latérale du dispositif de la figure 2a.

Le mode de réalisation selon l'exemple représenté sur les figures 2a et 2b peut correspondre à celui des figures 1a et 1b. En particulier, le mode de réalisation selon l'exemple des figures 2a et 2b peut comprendre les mêmes éléments et/ou fonctions.

Comme illustré en particulier sur la figure 2b, les pieds et/ou jambes peuvent être configurés pour être articulés chacun par des charnières 105. Les pieds et/ou jambes peuvent ainsi être configurés pour être mobiles afin que l'utilisateur puisse se déplacer et/ou marcher avec ses jambes 3 lorsqu'il porte le système de portage 100. Par conséquent, les pieds et/ou jambes peuvent supporter au moins une partie du poids du système porteur et/ou de l'équipement médical sur le sol, lorsque l'utilisateur se déplace.

En outre, les pieds et/ou les jambes peuvent ainsi être configurés pour permettre à un utilisateur de s'asseoir sur une chaise.

En outre, les pieds et/ou les jambes peuvent être configurés pour maintenir une position de l'utilisateur assise et/ou baissée sans chaise. Cette position peut être une position de travail (par exemple, si l'utilisateur examine un patient qui est assis ou couché), qui soulage les jambes et/ou le corps de l'utilisateur du poids du dispositif. Notamment, le maintien de cette position de travail permet à l'utilisateur de s'affranchir de la pénibilité liée aux contraintes de positions imposées par l'examen.

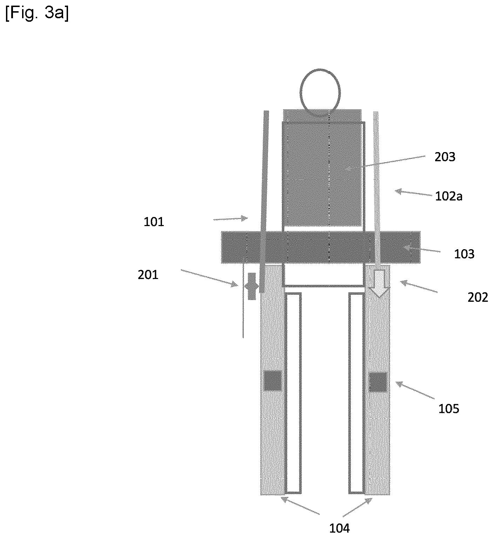

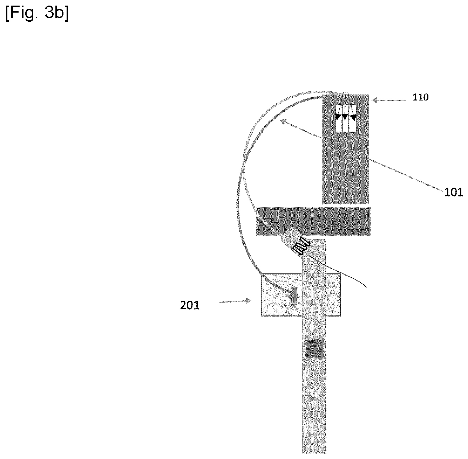

La figure 3a représente schématiquement une vue de face du dispositif des figures 1a, 1b et 2a, 2b dans une position de déplacement et/ou de stationnement. La figure 3b représente schématiquement une vue latérale du dispositif de la figure 3a.

Le mode de réalisation selon l'exemple représenté sur les figures 3a et 3b peut correspondre à celui des figures 1a et 1b. En particulier, le mode de réalisation selon l'exemple des figures 3a et 3b peut comprendre les mêmes éléments et/ou fonctions. Toutefois, dans l'exemple illustré, les éléments peuvent être dans une position rétractée, de sorte que le dispositif 10 peut être facilement transporté et/ou rangé/garé. Cette position peut également être utilisée lorsque l'utilisateur se déplace, par exemple lorsqu'il doit se rendre sur une zone d'intervention, lors d'une catastrophe, un accident, dans un service d'un hôpital.

Par exemple, le deuxième bras 101 peut être mobile de sorte que le dispositif d'écran 201 peut être déplacé de la position étendue à une position rétractée, dans laquelle le dispositif d'écran peut être plus proche et/ou adjacent à au moins un élément parmi le corps de l'utilisateur, le sac à dos et le système de ceinture.

De même, le premier bras 102a peut être mobile de sorte que la sonde 202 peut être déplacée de la position étendue à une position rétractée, où le porte sonde et/ou la sonde 202 peuvent être plus proches et/ou adjacents à au moins un élément parmi le corps de l'utilisateur, le sac à dos et le système de ceinture.

Dans l'exemple illustré, le premier et le second bras peuvent en particulier être adjacents au sac à dos 110 et au système de ceinture 103. Optionnellement, le premier et/ou le second bras peut comprendre des moyens de fixation configurés pour permettre la fixation du bras au système de ceinture et/ou à une jambe 104, en particulier en position rétractée.

La figure 4 représente schématiquement une vue de face d'un deuxième mode de réalisation d'un dispositif selon la présente divulgation comprenant un casque (par exemple un visiocasque, casque de réalité virtuelle) et/ou une interface utilisateur.

Le mode de réalisation selon l'exemple représenté sur la figure 4 peut correspondre à celui des figures 1a et 1b. En particulier, le mode de réalisation selon l'exemple de la figure 4 peut comprendre les mêmes éléments et/ou fonctions. Toutefois, dans cet exemple, le dispositif d'écran et le deuxième bras peuvent être remplacés par un visiocasque. En conséquence, l'utilisateur peut porter le visiocasque sur la tête. Le visiocasque peut communiquer avec l'unité de traitement 203 via un câble et/ou sans fil. En variante, il est également possible que le dispositif 10 comprenne une combinaison du dispositif d'écran et du visiocasque.

Le système de sac à dos et/ou de ceinture peut être configuré pour porter une unité de commande 205 avec une interface utilisateur. L'unité de commande peut permettre à l'utilisateur de contrôler le dispositif 10 et/ou l'équipement médical 200. Ceci peut notamment être utile, dans le cas où le dispositif 10 ne comprend pas de dispositif à écran tactile. L'unité de commande 205 peut comprendre par exemple un ou plusieurs boutons de commandes, interrupteurs, curseurs et éventuellement un trackpad.

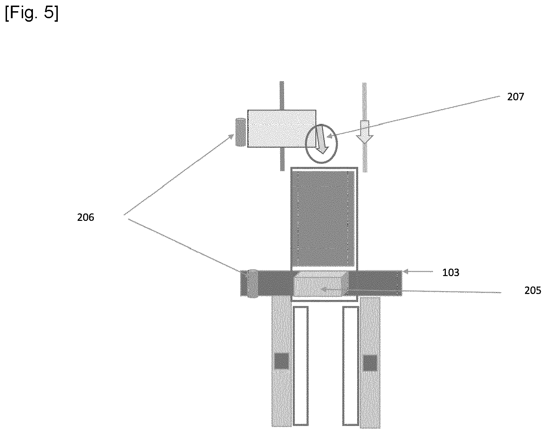

La figure 5 représente schématiquement une vue de face d'un troisième mode de réalisation d'un dispositif, selon la présente divulgation, comprenant des accessoires.

Le mode de réalisation selon l'exemple représenté sur la figure 5 peut correspondre à celui de l'une quelconque des autres figures. En particulier, le mode de réalisation selon l'exemple de la figure 5 peut comprendre les mêmes éléments et/ou fonctions. Toutefois, dans cet exemple, le dispositif 10, en particulier le système porteur 100, peut comprendre en outre un ou plusieurs accessoires. Les accessoires peuvent comprendre par exemple un flacon de gel 206 qui peut être fixé au système de ceinture et, en outre ou en variante, un flacon de gel 206 fixé au deuxième bras 101 portant le dispositif d'écran et/ou au premier bras 102a portant la sonde.

En outre, les accessoires peuvent comprendre une source lumineuse 207 configurée pour éclairer une zone située devant l'utilisateur, notamment de façon à éclairer le milieu à étudier. La source lumineuse peut être fixée de manière mobile de sorte que la zone éclairée puisse être ajustée en fonction des besoins, par exemple de la position du milieu. Par exemple, la source lumineuse peut être fixée de manière mobile au premier ou au second bras. Le dispositif peut également comprendre une pluralité de sources lumineuses, par exemple fixées à chacun des deux bras. Le dispositif peut également comprendre une unité de commande 205, comme par exemple décrit dans le contexte de la figure 4. L'unité de commande 205 peut en outre être configurée pour commander les accessoires. Par exemple, l'unité de commande peut comprendre un bouton pour allumer et éteindre la source lumineuse 207, et/ou pour régler son intensité, sa couleur, ....

La figure 6a représente schématiquement une vue de face d'un quatrième mode de réalisation d'un dispositif selon la présente divulgation. Le mode de réalisation selon l'exemple représenté sur la figure 6a peut correspondre à celui de l'une quelconque des autres figures. En particulier, il peut comprendre les mêmes éléments et/ou fonctions.

Toutefois, comme le montre l'exemple de la figure 6a, le deuxième bras 101 tenant le dispositif d'écran 201 peut également être relié au système de ceinture 103 (au lieu du sac à dos) et/ou s'étendre à partir de celui-ci. Par exemple, le deuxième bras peut être relié à l'avant ou à un côté d'une ceinture entourant la hanche de l'utilisateur. Un mode de connexion correspondant peut également s'appliquer au premier bras 102a.

En outre, comme le montre l'exemple de la figure 6a, le premier bras 102a peut également être configuré de manière à s'étendre le long du bras 5 de l'utilisateur et/ou à se déplacer avec lui. Par exemple, il peut être connecté au sac à dos et/ou s'étendre à partir de celui-ci dans une région de l'épaule de l'utilisateur. Dans cet exemple, le câble de sonde optionnel 202a peut être guidé le long du premier bras.

Dans un exemple, le premier bras 102a peut être recouvert d'un manchon flexible qui peut englober le bras de l'utilisateur par exemple en tissu, latex ou nitrile, et/ou il peut comprendre un matériel aseptisé, jetable ou non. Ce matériel peut par exemple être configuré pour protéger l'utilisateur et le patient contre des infections. En outre, le premier bras peut comprendre et/ou être recouvert d'une protection flexible configurée pour couvrir également la sonde et optionnellement aussi le bras de l'utilisateur. Par conséquent, la protection peut aussi protéger la main de l'utilisateur et la sonde contre des infections d'une part et le patient d'autre part. Dans un autre exemple, le premier bras 102a peut comprendre au moins deux éléments rigides reliés par une charnière. En conséquence, le premier bras peut être configuré pour supporter le poids de la sonde et/ou du câble de la sonde. Dans un autre exemple encore, le deuxième bras peut simplement consister en une pluralité de sangles (par exemple avec des attaches Velcro ou des aimants) configurées pour être attachées à une ou plusieurs positions du bras de l'utilisateur et pour maintenir la sonde et/ou le câble de la sonde. Notamment, le câble peut être situé le long et/ou dans le premier bras 102a.

Il est également possible que le second bras comprenne un gant et/ou une manche entourant l'avant-bras, configuré pour avoir un porte sonde sur le dos de la main de l'utilisateur. Ledit porte sonde peut être configuré pour maintenir la sonde dans une position telle que le capteur de la sonde s'étend dans la direction de l'avant-bras. En conséquence, l'utilisateur peut être en mesure d'acquérir des données à l'aide de la sonde en tenant la sonde (dépassant de l'avant-bras) près d'un milieu.

Selon un exemple, la sonde peut être fixée sur le dessus du poignet. Il est également possible que la sonde soit fixée de manière à ce qu'elle repose dans la main de l'utilisateur. Dans ce contexte, il est également possible que la sonde soit fixée sous l'avant-bras et qu'elle soit extensible/glissante vers l'avant afin que l'utilisateur puisse la saisir et la 'ranger' facilement au besoin. Les modifications du premier et du deuxième bras, telles que décrites dans le contexte de la figure 6a, peuvent également être combinées avec les modes de réalisation des figures précédentes, indépendamment les unes des autres (par exemple, uniquement la modification du premier bras, ou uniquement la modification du deuxième bras).

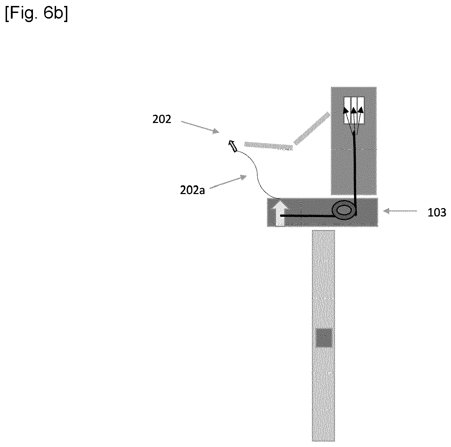

La figure 6b représente schématiquement une vue latérale du dispositif de la figure 6a avec des modifications optionnelles.

Le mode de réalisation selon l'exemple représenté sur la figure 6b peut correspondre à celui de l'une quelconque des figures 6a. En particulier, il peut comprendre les mêmes éléments et/ou fonctions.

Toutefois, comme le montre l'exemple de la figure 6b, le câble de sonde 202a peut également s'étendre à partir du système de ceinture 103 (par exemple une ceinture entourant la hanche de l'utilisateur) au lieu de s'étendre le long du premier bras 102a. Dans ce cas, le câble de sonde peut s'étendre d'un connecteur de l'unité de traitement vers le système de ceinture, et à l'intérieur du système de ceinture vers une ouverture sur le côté avant de l'utilisateur. Le câble peut être extensible hors de l'ouverture, par exemple au moyen d'une ou plusieurs bobines dans le système de ceinture.

Il est encore à noter que le deuxième bras 101 et le dispositif d'écran 201 sont omis sur la figure 6b simplement en raison d'une simplification de l'illustration. Le mode de réalisation selon l'exemple de la figure 6b peut éventuellement comprendre ces éléments.

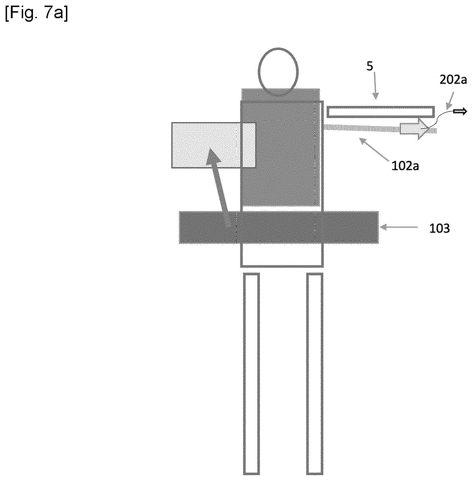

La figure 7a représente schématiquement une vue de face d'un cinquième mode de réalisation d'un dispositif selon la présente divulgation. Le mode de réalisation selon l'exemple représenté sur la figure 7a peut correspondre à celui de l'une quelconque des autres figures. En particulier, il peut comprendre les mêmes éléments et/ou fonctions.

Toutefois, comme le montre l'exemple de la figure 7a, les jambes/et ou les pieds peuvent être omis. A la place, le système porteur 100 et notamment le système de ceinture 103 peut être configuré pour supporter le poids du dispositif 10, notamment de l'équipement médical 200. En conséquence, le dispositif peut permettre une meilleure mobilité de l'utilisateur.

Dans un exemple, le dispositif 1 peut être configuré pour être fixé uniquement sur le haut du corps de l'utilisateur (par exemple à partir de la hanche de l'utilisateur et vers le haut de son corps).

Dans l'exemple illustré, le dispositif peut correspondre à celui de la figure 6a, avec des jambes et/ou des pieds omis. Grâce à cette configuration (décrite dans le contexte de la figure 6a), les éléments du dispositif (en particulier l'écran et la sonde) peuvent être disposés près du corps de l'utilisateur. Par conséquent, l'utilisateur peut supporter le poids de ces éléments avec moins d'effort et l'omission des jambes et/ou des pieds peut ainsi être compensée au moins en partie.

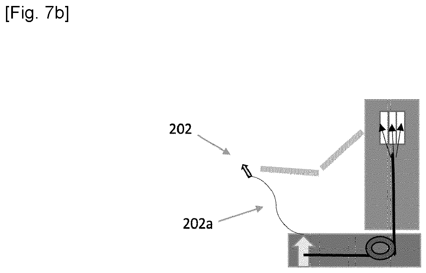

La figure 7b représente schématiquement une vue latérale du dispositif de la figure 7a avec des modifications optionnelles. Dans l'exemple illustré, le dispositif peut correspondre à celui de la figure 6b avec des jambes et/ou des pieds omis.

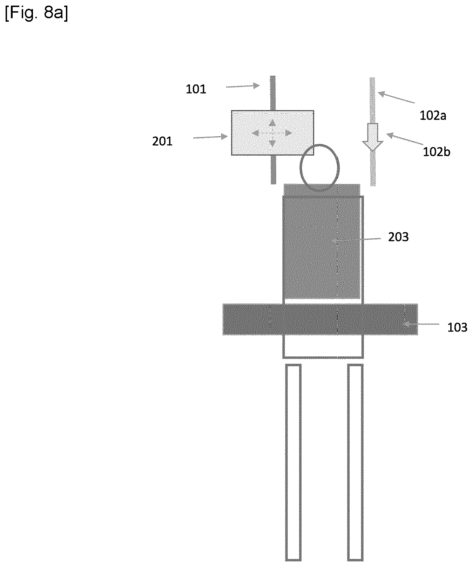

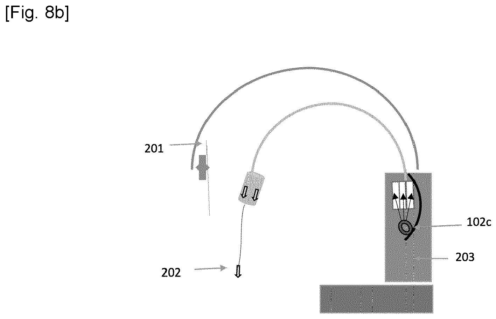

La figure 8a montre schématiquement une vue de face d'un sixième mode de réalisation d'un dispositif selon la présente divulgation. La figure 8b montre schématiquement une vue latérale du dispositif de la figure 8a. Le mode de réalisation selon l'exemple représenté sur la figure 8a peut correspondre à celui de l'une quelconque des autres figures 1a à 6b. En particulier, il peut comprendre les mêmes éléments et/ou fonctions.

Toutefois, comme le montre l'exemple de la figure 8a, les jambes/et ou les pieds peuvent être omis. A la place, le système porteur 100 et notamment le système de ceinture 103 peut être configuré pour supporter le poids du dispositif 10, notamment de l'équipement médical 200. En conséquence, le dispositif peut permettre une meilleure mobilité de l'utilisateur.

Dans un exemple, le dispositif 1 peut être configuré pour être fixé uniquement sur le haut du corps de l'utilisateur (par exemple à partir de la hanche de l'utilisateur et vers le haut).

Dans l'exemple illustré, le dispositif peut correspondre à celui de la figure 1a avec des jambes et/ou des pieds omis. Grâce à cette configuration (décrite dans le contexte de la figure 1a), le dispositif d'écran et/ou la sonde (ou le porte-sonde) peuvent être disposés dans une position confortable pour l'utilisateur. En d'autres termes, les deux éléments peuvent être disposés face à l'utilisateur. En particulier, le dispositif d'écran peut être disposé de telle sorte que l'affichage sur l'écran puisse être facilement vu par l'utilisateur, indépendamment de la position de l'utilisateur (par exemple debout, assis, en train de marcher, en changeant de position par rapport à un patient, etc.) En outre, la sonde (ou le porte-sonde) peut également être disposée de manière à ce que l'utilisateur puisse facilement l'atteindre et/ou la saisir, indépendamment de sa position.

Tous ces modes de réalisations et d'autres exemples tels que décrits ci-dessus sont donnés uniquement à titre d'exemple non limitatif, et peuvent être combinés et/ou modifiés selon la portée des revendications suivantes.

Une référence à un document de brevet ou à tout autre élément identifié comme étant de l'art antérieur ne doit pas être considérée comme une admission que le document ou l'autre élément était connu ou que les informations qu'il contient faisaient partie de la connaissance générale commune à la date de priorité de l'une quelconque des revendications.

## Revendications

1. Dispositif médical portable comprenant :
un équipement médical d'acquisition de données et un système porteur, dans lequel l'équipement médical comprend :
une unité de traitement configurée pour traiter les données acquises par une sonde associée avec l'unité de traitement,
et dans lequel le système porteur est configuré pour équiper le corps d'un utilisateur du dispositif avec l'équipement médical.

2. Dispositif médical portable selon la revendication 1, dans lequel l'équipement médical comprend en outre la sonde à ultrasons, et/ou l'unité de traitement comprend une batterie et/ou est configurée pour fonctionner de manière autonome à partir d'une alimentation électrique externe.

3. Dispositif médical portable selon la revendication 1 ou 2, dans lequel le dispositif médical comprend en outre une unité de commande avec une interface utilisateur configurée pour permettre à l'utilisateur de commander le dispositif médical.

4. Dispositif médical portable selon l'une quelconque des revendications précédentes, dans lequel
l'unité de traitement est configurée pour recevoir et/ou traiter des données externes d'un système externe.

5. Dispositif médical portable selon l'une quelconque des revendications précédentes, dans lequel
l'équipement médical comprend en outre un écran d'affichage et/ou un visiocasque, dans lequel
l'unité de traitement est configurée pour que le dispositif d'affichage et/ou le visiocasque affiche des informations basées sur les données traitées et/ou les données externes.

6. Dispositif médical portable selon l'une quelconque des revendications précédentes, dans lequel
le système porteur est configuré pour être ajusté au corps de l'utilisateur et/ou pour être installé sur le corps de l'utilisateur, et/ou
le système porteur comprend un sac à dos configuré pour porter l'unité de traitement.

7. Dispositif médical portable selon l'une quelconque des revendications précédentes, dans lequel
le sac à dos comprend un système de ceinture configuré pour être porté par le tronc de l'utilisateur et pour faire supporter au tronc de l'utilisateur au moins une partie du poids du système porteur et/ou de l'équipement médical.

8. Dispositif médical portable selon l'une quelconque des revendications précédentes, dans lequel
le sac à dos et/ou le système de ceinture est configuré pour porter la batterie et/ou l'unité de contrôle et/ou des accessoires configuré pour être utilisable en préparation et/ou pendant un examen médical.

9. Dispositif médical portable selon l'une quelconque des revendications précédentes, dans lequel
le système porteur comprend un premier bras configuré pour maintenir la sonde dans une position telle que la sonde puisse être saisie par une main de l'utilisateur.

10. Dispositif médical portable selon l'une quelconque des revendications précédentes, dans lequel
le premier bras comprend un porte sonde configuré pour tenir la sonde, et/ou
le premier bras est en outre configuré pour tenir et/ou contenir un câble reliant la sonde à l'unité de traitement.

11. Dispositif médical portable selon l'une quelconque des revendications précédentes, dans lequel
le câble est extensible hors du sac à dos et/ou hors du premier bras.

12. Dispositif médical portable selon l'une quelconque des revendications précédentes, dans lequel
le premier et/ou deuxième bras et/ou le porteur sonde est motorisé,
et l'unité de traitement est configuré pour contrôler un déplacement du premier et/ou deuxième bras et/ou du porteur sonde d'une manière automatique.

13. Dispositif médical portable selon l'une quelconque des revendications précédentes, dans lequel
le premier bras et/ou le porteur sonde comprend un capteur de localisation, et l'unité de traitement est configuré pour :
déterminer un déplacement de la sonde en fonction des données acquises par le capteur de localisation, et/ou
fusionner des données acquises par la sonde avec des données externes en fonction des données acquises par le capteur de localisation.

14. Dispositif portable à ultrasons selon l'une quelconque des revendications précédentes, dans lequel
le premier bras est mobile de telle sorte que :
la position déployée de la sonde est réglable, et/ou
la sonde peut être déplacée de la position étendue à une position rétractée, dans laquelle la sonde est plus proche et/ou adjacente à au moins un élément parmi le corps de l'utilisateur, le sac à dos et le système de ceinture.

15. Dispositif médical portable selon l'une quelconque des revendications précédentes, dans lequel
le système porteur comprend un deuxième bras configuré pour maintenir le dispositif d'affichage dans une position étendue par rapport à l'utilisateur, de sorte que les informations affichées puissent être accessibles à l'utilisateur et/ou le patient.

16. Dispositif médical portable selon l'une quelconque des revendications précédentes, dans lequel :
le deuxième bras est mobile de sorte que :
la position du dispositif d'affichage peut être ajustée et/ou
le dispositif d'affichage peut être déplacé de la position étendue à une position rétractée, où le dispositif d'affichage est plus proche et/ou adjacent à au moins un élément parmi le corps de l'utilisateur, le sac à dos et le système de ceinture.

17. Dispositif médical portable selon l'une quelconque des revendications précédentes, dans lequel :
le premier et/ou le deuxième bras est fixé de manière mobile au sac à dos et/ou au système de ceinture, et/ou
le premier et/ou le deuxième bras est configuré pour être maintenu par le sac à dos et/ou le système de ceinture.

18. Dispositif médical portable selon l'une quelconque des revendications précédentes, dans lequel :
le système porteur comprend au moins un pied et/ou jambe configuré pour supporter au moins une partie du poids du système porteur et/ou du dispositif médical sur un sol sur lequel se tient l'utilisateur.

19. Dispositif médical portable selon l'une quelconque des revendications précédentes, dans lequel :
le au moins un pied et/ou jambe est configuré pour être articulé et/ou mobile de sorte que l'utilisateur peut se mouvoir et/ou marcher, tandis que le pied et/ou jambe supporte au moins une partie du poids du système porteur et/ou de l'équipement médical sur le sol.

20. Dispositif médical portable selon l'une quelconque des revendications précédentes, dans lequel :
le au moins un pied et/ou jambe est configuré pour être associé aux membres inférieurs de l'utilisateur pour suivre le mouvement des membres inférieurs de l'utilisateur.

21. Le dispositif médical portable selon l'une quelconque des revendications précédentes, dans lequel :
le système porteur comprend ou est un exosquelette, dans lequel
l'exosquelette comprend au moins l'un des éléments suivants :
le sac à dos,
le système de ceinture,
le premier bras,
le deuxième bras, et
le au moins un pied et/ou jambe.
